# EUROPEAN PATENT SPECIFICATION

(11) **EP 2 203 070 B1**
(45) Date of publication and mention of the grant of the patent: **27.03.2013**
(21) Application number: 08807007.3
(22) Date of filing: 05.09.2008
(51) Int. Cl.: A23K 1/16, A23L 1/31, A23L 2/02, A61K 36/185, A23C 9/152, A23K 1/18, A23L 1/30, A23L 1/318, A23L 2/52, A61K 31/352, A61P 9/12, A23L 1/325

(54) **POMEGRANATE EXTRACTS, NUTRITIONAL PRODUCTS CONTAINING THEM AND THEIR USES**
GRANATAPFELEXTRAKTE, SIE ENTHALTENDE NÄHRMITTELPRODUKTE UND IHRE VERWENDUNGEN
PRODUITS NUTRITIFS COMPORTANT DES EXTRAITS DE GRENADE CONTENANT DES TANINS ELLAGIQUES ET LEUR UTILISATION

(30) Priority: 07.09.2007 EP 07017570; 07.03.2008 EP 08004242
(43) Date of publication of application: 07.07.2010
(73) Proprietor: Probelte Pharma, S.A., 30100 Espinardo Murcia (ES)
(72) Inventor: LÓPEZ MÁS, José A., E-30840 Alhama de Murcia Murcia (ES); STREITENBERGER, Sergio A., E-30120 El Palmar Murcia (ES); PEÑALVER MELLADO, Marcos, E-30007 Murcia Murcia (ES); PEDREÑO LÓPEZ, Yolanda, E-30004 Murcia Murcia (ES); MARTINEZ ORTIZ, Pedro, E-30150 La Alberca Murcia (ES)
(74) Representative: Gislon, Gabriele
(86) International application number: PCT/IB2008/002316
(87) International publication number: WO 2009/031023

(56) References cited:
- EP-A- 1 293 130
- EP-A- 1 967 078
- WO-A-02/056899
- WO-A-2006/127832
- JP-A- 2005 154 323
- US-A1- 2006 211 635
- SEERAM N ET AL: "Rapid large scale purification of ellagitannins from pomegranate husk, a by-product of the commercial juice industry" SEPARATION AND PURIFICATION TECHNOLOGY, ELSEVIER SCIENCE, AMSTERDAM, NL, vol. 41, no. 1, 1 January 2005 (2005-01-01), pages 49-55, XP004608865 ISSN: 1383-5866
- DATABASE WPI Week 200370 Thomson Scientific, London, GB; AN 2003-735551 XP002467614 & JP 2003 102431 A (PERUSHAZAKURO YAKUHIN KK) 8 April 2003 (2003-04-08)
- NEGI P S ET AL: "ANTIOXIDANT AND ANTIBACTERIAL ACTIVITIES OF PUNICA GRANATUM PEEL EXTRACTS" JOURNAL OF FOOD SCIENCE, WILEY-BLACKWELL PUBLISHING, INC, US, vol. 68, no. 4, 1 May 2003 (2003-05-01), pages 1473-1477, XP009016005 ISSN: 0022-1147
- SINGH R P ET AL: "Studies on the Antioxidant Activity of Pomegranate (Punica granatum) Peel and Seed Extracts Using in Vitro Models" JOURNAL OF AGRICULTURAL AND FOOD CHEMISTRY, AMERICAN CHEMICAL SOCIETY, WASHINGTON, US, vol. 50, no. 1, 17 November 2001 (2001-11-17), pages 81-86, XP002251850 ISSN: 0021-8561
- LANSKY ET AL: "Punica granatum (pomegranate) and its potential for prevention and treatment of inflammation and cancer" JOURNAL OF ETHNOPHARMACOLOGY, ELSEVIER SCIENTIFIC PUBLISHERS LTD, IE, vol. 109, no. 2, 22 December 2006 (2006-12-22), pages 177-206, XP005812637 ISSN: 0378-8741

## Description

### Field of the invention.

The present invention relates to the use of pomegranate extracts containing ellagitannins, to prepare nutritional products containing these added pomegranate extracts and to the use of both pomegranate extracts and the nutritional products functionalized with pomegranate extracts for prevention or treatment of cardiovascular diseases (CVD), plaque build-up in the arteries, arterial hypertension, and metabolic syndrome. According to that, pomegranate extract derived from whole pomegranate fruit is added to beverages and human or veterinary food products, which results in an increase of the level of anti-oxidants, particularly of ellagitannins and more particularly of punicalagins. More particularly, the invention relates to the functionalization or fortification of nutritional products to be used as a source of punicalagins for prevention or treatment of cardiovascular diseases (CVD), plaque build-up in the arteries, arterial hypertension, and metabolic syndrome., thanks to the nutritional supply of a punicalagins rich composition.

According to the supplement published by the British Heart Foundation: European cardiovascular disease statistics, 2005 edition, CVD are the main cause of death in Europe: accounting for over 4.35 million deaths each year. Coronary heart disease (CHD) by itself is the single most common cause of death in Europe: accounting for 1.95 million deaths in Europe each year.

Total costs of CVD amounts to 169 billion euros, of which 105 billion euros are for treating CVD in the European Union and 64 billion euros are due to lost productivity and the cost of informal care.

Plaque builds up in the arteries, also called atherosclerosis is the main cause of CVD and the most frequent cause of CHD. Atherosclerotic plaque builds up in the arteries is a common disorder of the arteries. It occurs when fat, cholesterol, and other substances build up in the walls of arteries and form hard substances called plaque.

Eventually, the plaque deposits can make the artery narrow and less flexible. This makes it harder for blood to flow. If the coronary arteries become narrow, blood flow to the heart can slow down or stop, causing chest pain (stable angina), shortness of breath, heart attack, and other symptoms.

Pieces of plaque can break apart and move through the bloodstream. This is a common cause of heart attack and stroke. Blood clots can also form around the plaque deposits. Clots block blood flow. If the clot moves into the heart, lungs, or brain, it can cause a stroke, heart attack, or pulmonary embolism.

It has been demonstrated that arterial hypertension, high levels of triglycerides and total cholesterol in the blood, and smoking are factors that contribute to the development of this affection. In recent years, researchers have found that some of these risk factors cluster together in certain people. This clustering of risk factors is known as metabolic syndrome.

People with metabolic syndrome have a clustering of the following risk factors:
- Central obesity, meaning extra weight in the abdominal (stomach) area.
- Problems in digesting glucose (glucose intolerance). Patients with metabolic syndrome usually have hyperinsulinemia or type 2 diabetes.
- High levels of low-density lipoprotein (LDL) and triglycerides in the bloodstream.
- Low levels of high-density lipoprotein (HDL) in the bloodstream.
- High blood pressure (hypertension).

There is still a lot to be learned about metabolic syndrome, but doctors know that people with metabolic syndrome have an increased risk of CVD.

### Background of the invention.

Numerous studies have demonstrated that in vivo oxidation of LDL plays a central role in the development of atherosclerosis (Knight, 1995; Witzum, 1994).

In both, laboratory and clinical studies, pomegranate shows promising results in preventing cardiovascular diseases. Scientists provide evidences about pytochemicals from pomegranate work through several mechanisms: by inhibiting the oxidation of LDL and supporting the synthesis and activity of nitric oxide (De Nigris et al, Nitric Oxide. 2006), reducing oxidative stress and inflammatory damage in blood vessels (De Nigris et al. Cardiovasc. Res. 2007; Rozenberg et al. Atherosclerosis. 2006), decreasing activity of angiotensin converting enzyme (Aviram et al, Atherosclerosis. 2001). Mounting evidence suggest that compounds in pomegranate known as punicalagins are cardioprotective by virtue of their powerful antioxidant and anti-inflammatory affects. M. Shiner et al., Atherosclerosis 195, 2007, report the effects of up-regulation of Paraoxonase2 by punicalagin and gallic acid; paraoxonase 2 was shown to protect macrophages against oxidative stress, through a yet unknown physiological role.

Further studies have shown that pomegranate juice has the higher antioxidant properties than any other drink, such as blueberry juice, cranberry juice, green tea or red wine. Punicalagins α and β anomers are predominant ellagitannins in pomegranate juice, and the major responsible of antioxidant properties of the juice. Nevertheless, research pointed out that combination of punicalagins α and β with other ellagitannins such as ellagic acid and its glycosidated derivatives and other minor pomegranate ellagitannins produces and important synergistic effect enhancing healthy benefits of the juice.

***Rapid large scale purification of ellagitannins from pomegranate husk,a by-product of the commercial juice industry*** (Seeram et al. Separation and Purification Technology 41 (2005) 49-55, January 2005) discloses a method to isolate large amounts of total pomegranate tannins (TPT) from fruit husk which contains high levels of punicalagin. The method uses water and low carbon alcohols as eluants. Using disclosed method, evaluation of TPT showed that it contains the major fruit husk ellagitannin (ET), punicalagin (80-85% w/w) and ellagic acid (EA; 1.3% w/w) and various amounts of further compounds such as punicalin and EA-glycosides (hexoside, rhamnoside and pentoside).

A corresponding teaching is disclosed by US 2006/211635, by the authors of the above publication.

A review of the most recent studies is found in Lansky et al., Journal of Ethnopharmacology 109, 2007, 177-206. The review stresses the potential of the materials obtained from pomegranate, especially the oily phase of the seed, for treatment or prevention of cancer. However, the review concludes by stating that though inconclusive, the studies reported suggested further study, including clinical trials of properly designed pharmaceutical products.

Punicalagins α and β anomers are mainly present in pomegranate husk, and they pass to the primary juice during pressing for juice extraction. Punicalagins α and β have the following formula:

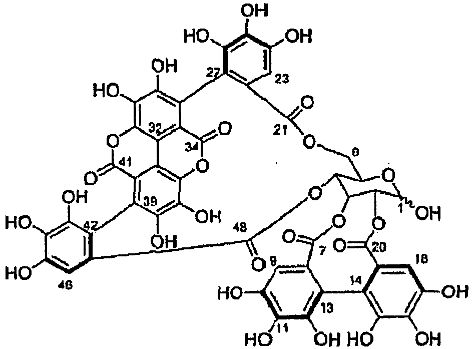

The studies generated a number of patent applications.

WO02/056899 relates to compositions and methods for reducing oxysterols in the blood and normalizing cholesterol and blood pressure. This patent discloses compositions that preferably comprise: Noni (Morinda Citrifolia) extract, red wine extract, prune extract, blueberry extract, pomegranate extract, apple extract, and an enzyme mixture. Patent claims a method to maintain or reduce oxysterols in blood comprising the step of administering the above mentioned composition wherein said pomegranate extract comprises about 40 % ellagic acid and about 60 % polyphenols. Moreover, when free ellagic acid is administrated orally, it is poorly absorbed.

US 2006/0211635 discloses purification of pomegranate ellagitannins from fruit husk, a by-product of the commercial juice industry. This process has the drawbacks of requiring the use, for elution of ellagitannins adsorbed onto a resin surface, of organic solvents, possibly toxic as methanol, and of generating an extract than doesn't contain several phytochemicals exclusively present in the pomegranate juice fraction, when in fact the juice is the product consumed by the people, to which recent studies attribute the healthy benefits obtainable from pomegranate.

WO2006/12783 discloses a method to produce a pomegranate extract from by-products of the juice industry. The process described, includes several thermical treatments where the extract is subjected to heating (2 h at 43-71 °C and pasteurization). Pomegranate juice pasteurization produces free ellagic acid trough degradation of larger ellagitannins, such as punicalagins, diminishing the health benefits of drinking the juice. These steps generate several by-products that are difficult to remove and, in addition, the use of pomegranate solids coming from the juice industry generates an extract than doesn't contain some phytochemicals exclusively present in the pomegranate juice fraction. For this reason WO2006/127832 discloses the combination of pomegranate extract and juice, which in fact provides a broad spectrum of the phytochemicals present in the whole pomegranate, but has the disadvantage of the high caloric value due to the sugars present in both the juice and the extract. Additionally, a high sugar content makes difficult the industrial processing of such extracts because the purification of the punicalagins is difficult and the solid extracts stick to the walls of the drying equipments. The patent claims related to beverages and food products comprising pomegranate extract are referred to extracts prepared from pomegranate solids selected from the group consisting of the pericarp, inner membrane and seeds but didn't refer to extracts prepared from whole pomegranate fruits.

US Application 20070178180 relates to pomegranate extracts and methods of using thereof, and specifically to methods of using pomegranate extracts for causing regression in lesions due to arteriosclerosis in humans. This document presents a human study with 19 patients with carotid artery stenosis (CAS). Ten of these patients consumed one glass of pomegranate juice every day during 12 months. Authors of the document conclude that results of the human study demonstrate statistically significant anti-atherogenic effects of pomegranate juice consumption in patients with CAS. Both the pomegranate juice used and the pomegranate extracts prepared according the above mentioned application are prepared without any specific purification step.

US Application 20020012710 relates to pomegranate products useful in improving health. Patent claims a method for use the pomegranate seed oil, comprising administering the pomegranate seed oil to a subject to exert an effect on a physiologic process. Into such effect it is included an antioxidant effect. Additionally, this patent claims a method of retarding the process of atherosclerosis, comprising administering fermented pomegranate juice to a patient.

Pomegranate extract was also used to protect from oxidation beverages containing acid milk, as discussed in EP 1 293 130; here, the pomegranate extract can be obtained by reflux in water, filtration, treatment with charcoal or from water/ethanol. The concentration of pomegranate extract in the beverage is within the range of 10 ppm to 50 ppm. There is no indication of the extract composition.

Summarizing, the above mentioned techniques provide pomegranate products that in spite of providing benefits to the health, are still too crude for certain technological applications of the functional food and beverage industry, which supposes some disadvantages such as:
- Regular consumption of the juice or the crude extracts supposes the uptake of a high caloric value due to the sugars present in both. ***Composition of pomegranate juice*** (Seeram et al. J. Nutr. 136:2481-2485, October 2006). A serving of pomegranate juice (PJ) available for human consumption in single strength form (240 mL) contains 34 g sugars, 35 g total carbohydrates, 30 mg sodium, 430 mg potassium, 0% of RDA of vitamin C and vitamin A, 4% calcium, and 2% iron (Pom Wonderful). PJ contains the following polyphenols: anthocyanins, 387 mg/L; punicalagins, 1561 mg/L; ellagic acids, 121 mg/L; and other hydrolyzable tannins, 417 mg/L.
- Use of organic solvents to obtain some of the above mentioned pomegranate extracts.
- Standardization of some of the above mentioned pomegranate extracts is very far from the natural profile of phytochemicals (e.g. ellagitannins) existing in the natural pomegranate juice obtained from processing of the whole pomegranate fruit. It is to say for instance, some extracts have been standardized to 40 % ellagic acid instead to punicalagins in comparison to the leading commercially available pomegranate juice containing normally lees than 0,1 % ellagic acid and about 0.2 % punicalagins.
- Such products are not completely water soluble, specially the extracts enriched in ellagic acid, meaning that its incorporation in beverages will produce turbidity making the product lees attractive for the consumers.
- free ellagic acid is poorly absorbed when administered orally.

In addition, punicalagins content is not standardized in most of the pomegranate products above mentioned.

### Summary of the invention.

It is an aim of the present invention to solve the above mentioned problems and to provide pomegranate extracts, and nutritional products containing said extracts, the extracts containing ellagitannins and having a composition such as to impart to the food in which they are incorporated the required health beneficial effects upon consumption of the food.

Such aim is achieved by means of the present invention that relates to the use of pomegranate extracts according to claim 1 and to functional foods and beverages with pomegranate extracts according to claims 4.

Suitable extracts for preparing nutritional products are pomegranate extracts comprising punicalagins, ellagic acid and sugars, wherein the ratio (w/w%) punicalagin/ellagic acid is in the range of 10/1 to 35/1, the ratio (w/w%) punicalagin/sugars is within the range of 10/1 to 50/1, the total sugar content is 2 % (w/w) or less and also having a total phenols content of at least 30 % w/w, (expressed as gallic acid equivalent), and a water solubility of at least 7 % w/v (30 g per litre).

Preferably, a suitable pomegranate extract will have a punicalagins content of at least 30 % w/w, an ellagic acid content of 2 % (w/w) or less, a total phenols content of at least 30 % w/w, (expressed as gallic acid equivalent), a water solubility of at least 7 % w/v (70 g per litre), a total sugar content of 2 % (w/w) or less and a residual organic solvent content of less than 1 ppb.

More preferably the extract will have a water solubility of at least 10 % w/v (100 g per litre), and a total sugar content of not more than 1 % (w/w).

Said pomegranate extract can comprise a soluble fiber having a beta-glucan (dry basis) content of at least 30 %. Said soluble fiber is preferably selected among oat beta-glucan rich soluble fiber, barley beta-glucan rich soluble fiber, or their mixtures.

It was found that the above disclosed extracts are exceptionally suitable to be used for maintaining normal levels or reducing until normal levels both the concentration of cholesterol constituents in the blood and the arterial blood pressure and more generally to provide the beneficial properties and effects mentioned in the above discussed studies and patent applications, avoiding non-pleasant alterations of properties of the fortified foods such us turbidity of the fortified beverages and increase of the caloric content. Additionally, the absence of even traces of organic solvents in the extracts of the invention is advantageous both from the economical, and health points of view when compared with other pomegranate extracts containing residual solvents.

The above mentioned extracts are obtainable by means of the process disclosed in co-pending patent applications EP07004765.9 and EP08004242.7. The process therein disclosed and claimed comprises the steps of carrying out an extraction of the entire blended fruits in water at a temperature within the range of 4°C to 30°C, preferably of 8°C to 25°C, and at a pH within the range of 3.5 to 5.0. The extraction is carried out in a mill and the duration of said extraction step is within the range of 15 to 150 minutes. The extraction mixture is then treated to remove solids and to obtain a clarified aqueous solution that is loaded in a chromatographic column of an adsorbent non-ionic resin; the products retained in the chromatographic column are eluted by shifting the pH with a basic solution, and displacing the products with demineralised water. Preferably, said shifting of the pH is carried out with a sodium bicarbonate elution buffer solution.

The liquid product eluted from the resin is preferably concentrated by nanofiltration and water is removed until the product is in a solid form. By means of this method it is possible to obtain a product with a very low sugar content. Such a product was not known in the art, as per the above discussion of prior art. Still according to the copending patent EP08004242.7, in one embodiment the pomegranate extracts of the invention, are concentrated and dried in the presence of soluble fibers, especially oat and barley beta-glucan rich soluble fibers. These soluble fibers act as carriers and are provided in a preferred amount of at least 30%. Preferably the soluble fibers have a beta-glucan (dry basis) content of at least 30 % and within the range of 30% to 60%. Priority of EP08004242.7 for this feature and the features not recited in prior application EP07004765.9 is claimed by the present application.

The process of production of such solvent-free natural pomegranate extracts, discussed in co-pending patent applications EP070047565.9 and EP08004242.7 is simple, effective, and not expensive, providing both water soluble punicalagins, rather than solvent soluble ellagic acid, and a broad spectrum of different natural organic chemicals which are present in the whole pomegranate fruit and useful to improve activity of the main compounds of the extract.

An aspect of the invention relates to the nutritional products obtained from foods (including beverages) by addition of said pomegranate extract, possibly combined with food grade soluble fibers. These products are obtained by incorporating and mixing the pomegranate extract with the food product to be functionalized. A further aspect of the invention relates to the uses of the extract according to claims 11 and 12.

Another aspect of the invention relates to the use of the nutritional products, functional foods and beverages, for prevention or treatment of one or more conditions selected from cardiovascular diseases (CVD), plaque build-up in the arteries, arterial hypertension, and metabolic syndrome.

The compositions of natural pomegranate extracts suitable for the uses of the invention are summarized in the following paragraph for better understanding of the present invention.

Compositions as obtainable from the above mentioned applications are ellagitannins containing products in liquid or solid form and are characterized by having the following characteristics:
- a punicalagins content (as % w/w) of not less than 30%,
- an ellagic acid (free acid) content (as % w/w) of not more than 2%,
- a total phenol content (as % w/w gallic acid equivalent) of not less than 30%,
- a solubility in water (as % w/v) of not less
than 7% and more preferably of not less than 10%.
- a residual organic solvent content that is not detectable or in any case less than 1 ppb (i.e. the extract is free from organic solvents).
- a total sugar content of not more than 2% (w/w) and preferably of not more than 1% (w/w).
- a content of other hydrolizable tannins of not less than 8% (w/w).
- a content of anthocyanins of 0.3% (w/w) or less, preferably 0.2% or less.

In these compositions, the ratio (w/w%) punicalagin/ellagic acid is in the range of 10/1 to 35/1, preferably of 20/1 to 35/1 and the ratio (w/w%) punicalagin/fruit sugars is within the range of 10/1 to 50/1, prefererably of 30/1 to 50/1. In fact, the extracts contain almost no sugars (only 0,06 % of the original sugars are present in the extract) and almost no anthocyanins (only 0,96 % of the originally present anthocyanins), when compared to the pomegranate juice. With total sugar content it is meant the sugars deriving from the pomegranate juice; mainly "fruit sugars" i.e. glucose, fructose. Amounts are e.g. determined by HPLC.

The typical composition of the pomegranate extract and of the relevant juice composition required to administer the same amount of punicalagins than 1 glass (i.e. one serving or 240 mL of pomegranate juice, i.e. PJ) are summarized in the following table. Column 2 gives the composition of the amount of pomegranate extract that is required to provide 374,6 ppm of punicalagins, corresponding to the amount of punicalagins present in one serving (240 ml) of Pomegranate Juice (PJ).

Column 3 shows for each compound the percent of the amount still present in the extract with respect to the original PJ; in other words it shows that 100% of the punicalagins is recovered and 0.06% of the sugars originally present in the juice is present in the extract. As mentioned, less than 0.3%, usually less than 0.2% of original antocyanins are present in the extract. Data of the average composition of Pomegranate Juice are previously disclosed at page 6 of this application.

**TABLE A - composition table**

| | *Pomegranate extract* | *Composition of an amount of Pomegranate extract equivalent to 240 mL of PJ* | *Relative compound composition extract*/*juice.* |
|---|---|---|---|
| *compound* | *Amount, % (w*/*w)* | *Amount, ppm* | *Amount, %* |
| anthocyanins | 0,05 to 0,20 | 0,9 | 0,96 |
| punicalagins | 30,0 to 60,0 | 374,6 | 100 |
| ellagic acid | 0,5 to 5,0 | 21,4 | 73,72 |
| other hydrolysable tannins | 8,0 to 15,0 | 107,0 | 106,95 |
| sugars | < 3,0 | 20,0 | 0,06 |
| organic solvents | not detectable | not detectable | -- |

The above quantities were obtained by analysis of pomegranate extract samples to determine anthocyanins (spectrophotometric method), punicalagins, ellagic acid and other hydrolysable tannins (HPLC method), sugars (HPLC method) and organic solvents (GC method).

As previously mentioned, it was surprisingly found that by using pomegranate extracts that follow the above mentioned ratios, it is possible to fortify food products (including beverages) without jeopardizing the organoleptic properties of the food products and it is possible at the same time to impart to the fortified food (i.e. to the nutritional product) the required health beneficial effects.

This result-is surprising also because, as mentioned above, very little antocyanins are present in the extract, due to their loss in extraction.

Therefore, in the nutritional products object of the invention the ratio (w/w%) punicalagin/ellagic acid is in the range of 10/1 to 35/1, preferably of 20/1 to 35/1 and the ratio (w/w%) punicalagin/added pomegranate sugars is within the range of 10/1 to 50/1, preferably of 30/1 to 50/1. In particular, it should be noticed that the ratio of punicalagin vs. fructose in the extract, ad, consequently in the food products according to the invention (e.g. those that are initially free of fructose), is within the range of 20/1 to 100/1, preferably 60/1 to 100/1.

A further advantageous aspect is that the pomegranate extracts of the invention, are combined with food grade soluble fibers, especially oat and barley beta-glucan rich soluble fibers. These soluble fibers could act as carriers used for the drying process or simply be blended with the pomegranate extract obtained by drying without any added carrier, and are provided in a preferred amount of at least 30%. Preferably the soluble fibers have a beta-glucan (dry basis) content of at least 30 % and within the range of 30% to 60%.

By using natural extracts according to patent applications EP07004765.9 and EP08004242.7 it is avoided any trace of organic solvents in the final product as well as the high caloric value due to the sugars present in other pomegranate products, especially in view of their use for prevention or treatment of cardiovascular diseases (CVD), plaque build-up in the arteries, arterial hypertension, and metabolic syndrome.

The invention process of preparing a nutritional (or fortified) food (here the word food is including beverages) with pomegranate extract optionally with soluble fiber, comprises the steps of incorporating and mixing the pomegranate extract, combined or not with soluble fibers, with the food product to obtain a so-called functional or functionalized nutritional product. The addition of the pomegranate extract to the nutritional product is obtained by mixture and homogenisation according to the technological process of manufacturing each nutritional product which will be functionalized.

Functionalization (i.e. fortification) of solid food products for human or animal consumption comprises the step of adding pomegranate extract combined or not with soluble fibers, usually as early as possible, in the production process to facilitate its incorporation and homogeneous distribution whether the ingredients are pre-mixed with other dry ingredients or dispersed in water, according to the known art.

Functionalization (fortification) of beverages comprises the step of incorporating and dissolving the pomegranate extract combined or not with soluble fiber during the technical steps of beverage preparation. In a general way, pomegranate extract powder and optionally soluble fiber and further hydrophilic components are added, in the weight ratio desired, to the beverage and completely dissolved, under very controlled parameters of temperature and agitation. This step is preferably carried out before the beverage is pasteurized or food acids and/or food preservatives are added, to avoid spoilage.

In the context of the present invention, by the expression "nutritional product" it is understood any food product (including beverages, salt, sugar and seasoning) for human or animal consumption that incorporates an amount of a pomegranate extract.

According to the present invention, in the nutritional product, i.e. the food product including the pomegranate extract, the ratio (w/w%) punicalagin/ellagic acid is in the range of 10/1 to 35/1, preferably of 20/1 to 35/1 and the ratio (w/w%) punicalagin/added fruit sugars is within the range of 10/1 to 50/1, preferably of 30/1 to 50/1. The nutritional products of the invention preferably comprise a concentration of the pomegranate extract of the invention equivalent to a punicalagins content ranging from 0.005 % to 5 % (w/w). More generally, the nutritional product of the invention comprises an amount of pomegranate extract as above defined that will result in an uptake by the consumer of at least 10 mg of punicalagins per day; in other words, the nutritional product will provide at least 5 mg of punicalagins per serving.

In the context of the present invention, the expression "serving" is to be understood as meaning:
- a helping, i.e. an individual quantity of food or drink taken as part of a meal, or
- for one food, the amount a person, or an animal, would be likely to eat during a meal.

The above are the standard meaning used when assessing e.g. the calories content of a food and are also called "Common Measure". A list of the servings or common measures can be found e.g. in the "USDA National Nutrient Database for Standard Reference, Release 20, Nutrient List", available from the web site of the United States Department of Agriculture, Agricultural Research Service, Nutrient Data.

A preferred range comprises an amount of extract equivalent to a punicalagins content in the nutritional product ranging from 0.01 to 0.1 % (w/w).

Nutritional products containing this amount of added pomegranate extract can be: fruit-flavoured beverages, orange flavoured beverages, lemon-lime flavoured beverages, root beer, cola, fruit juices, fruit-flavoured, or fruit-containing beverages, vegetable juices or vegetable containing beverages, sport drinks, energy drinks, flavoured water, beverages comprising a dairy component, milk, milk shake, fermented milk, flavoured milk horchata, beer, beer-type sparkling drink, wine, wine-type drink, coffee, coffee-based beverages, tea, tea-based beverages, infusions, to name a few, technologically modified derivatives of the above mentioned beverages or mixtures of two or more of the same.

Another preferred range comprises an amount of extract equivalent to a punicalagins content ranging from 0.02 % to 0.20 % (w/w). Nutritional products containing this amount of added pomegranate extract can be:
- dairy product such as, yogurts, ice creams, cheeses.
- vegetable, marine or fish oils obtained from various sources such us olive (extra virgin olive oil, virgin olive oil, lampante olive oil, refined olive oil, crude olive-pomace oil, refined olive-pomace oil), sunflower, corn, soya, flax seed, almond, canola, safflower, palm, coconut, rapeseed, algae, krill, menhaden, anchovy, tuna.
- meat or poultry product such us, chicken, turkey, duck, pork, beef, prepared as sausages, smoke-dried or cold meat, such as cured ham, boiled ham, smoked ham, mortadella, salami, pâté, canned precooked meats.
- a bakery product or a pasta-based product such us breads, bagels, biscuits, cookies, cakes, pastries, pies, macaroni, spaghetti.
- a vegetable or fruit conserve such as canned tomatoes, canned artichokes, canned pineapple, canned peach, jams, marmalades, jellies, pickles.
- a canned seafood or canned fish such as shrimp, lobster, squid, crab, mussel, cockle, tuna, sardine.
- a snack or sweet such as almonds, peanuts, pistachios, walnuts, popcorns, chocolates, chewing gums, candies.
to name a few, technologically modified derivatives of the above mentioned food products or mixtures of two or more of the same.

Another preferred range comprises an amount of extract equivalent to a punicalagins content ranging from 0.025 % to 0.25 % (w/w). Nutritional products containing this amount of added pomegranate extract can be: pet food such us canned wet foods, dry pet foods, semi-moist foods, snacks, to name a few, technologically modified derivatives of the above mentioned pet foods or mixtures of two or more of the same.

The taste, flavour and in general organoleptic properties of the food is not modified by addition of the extract according to the present invention.

According to the invention, the extract is also added to salt in an amount of 0.5 % to 5 % (by weight) and to sugar (preferably with soluble fibers), in an amount of 0.2 % to 2 % (by weight). The low content of sugars of the extract according to the invention makes it particularly appealing to add the extract to seasonings (e.g. balsamic vinegar), salt, sugar and sweeteners (e.e. aspartame), their flavour being substantially not modified by the extract.

Finally a third aspect of the invention relates to the use of the nutritional products of the invention, functionalized with pomegranate extract combined or not with soluble fiber, for prevention or treatment of cardiovascular diseases (CVD), plaque build-up in the arteries, arterial hypertension, and metabolic syndrome. The uptake of functional foods and beverages with pomegranate extract combined or not with soluble fiber is preferable in accordance with a predetermined regimen, which preferably would be 2-3 servings daily, and may be over an extended period of time as a chronic treatment, and could last from one year or more, including the life of the consumer.

According to the invention, the recommended daily dose of a functional nutritional product containing the amount of the pomegranate extract of the present invention combined or not with soluble fiber, must contribute in sufficient quantity to achieve the intended purpose, when consumer take 2-3 servings per day in accordance with a predetermined regimen. In one embodiment, punicalagins are present in the functional nutritional product composition in an amount within the range of 5 mg to 5000 mg per serving, preferably 15 to 500 mg and more preferably of 40 mg to 200 mg per serving.

According to another preferred embodiment of the present invention, the fortified, i.e. functionalised, nutritional product contains the above mentioned extracts and hydroxytyrosol. Preferably, the hydroxytyrosol is obtained by extraction according to co-pending patent applications EP-A-07001791 filed 26.01.2007 and PCT/IB2008/000173 filed on 28.01.2008 in the name of the present applicant.

### Brief description of the drawings.

The invention will now be further disclosed in greater detail with reference to the enclosed following non-limiting examples and drawings wherein:
- figures 1-3 are graphs showing the linear correlation between the antioxidant capacity of different nutritional products obtained according the present invention (see examples 1 to 5) and its content in punicalagins measured by HPLC as a result of its fortification with increasing quantities of pomegranate extracts obtained according the present invention.
- Figures 4 is a graph showing the effect of pomegranate extract on rat atherogenic index (total cholesterol / HDL cholesterol).
- Figure 5 is a graph showing the effect of pomegranate extract on rat systolic blood pressure.

- Figure 6, is a graph showing the effects of administration of the pomegranate extract in combination with soluble fiber according to the present invention, on rat atherogenic index (total cholesterol / HDL cholesterol).
- Figure 7 is a graph showing the effect of the pomegranate extract in combination with soluble fiber on rat body weight (BW).

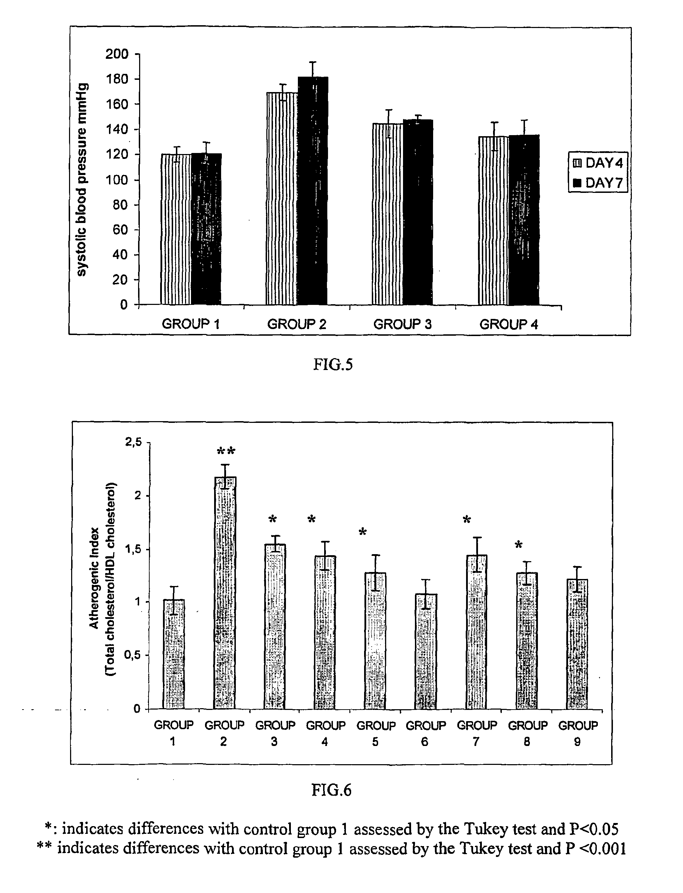

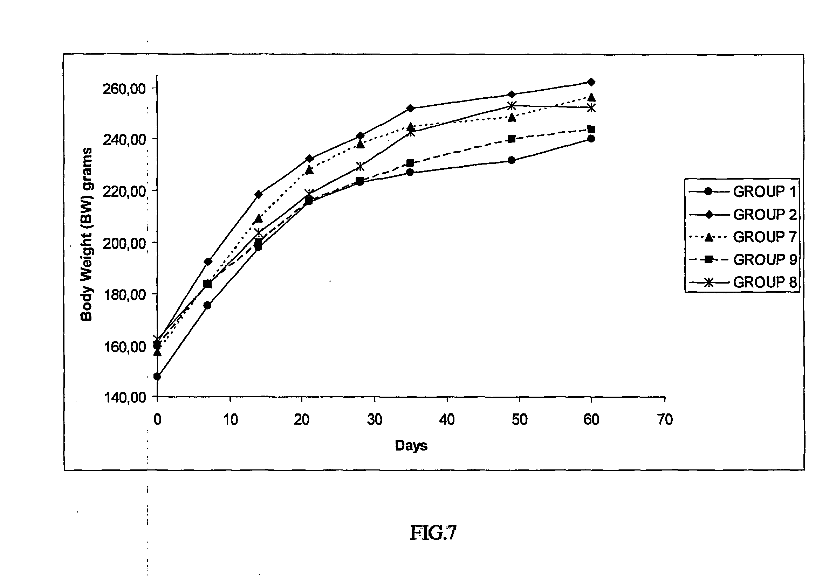

Figures 6 and 7 are related to examples 8a and 8b respectively, where the animal groups were organized according to Table 4 showed at the example 8a.

### Description of preferred embodiments.

### EXAMPLE 1

### Preparation of a functional orange juice with pomegranate extract

The product was prepared from a concentrated orange juice by addition of water and water soluble ingredients. Then, pomegranate extract was added and mixed and the resulting product was pasteurised and homogenized. Finally, the product was cooled and packaged. The same procedure is followed also when other ingredients are used such as ascorbic acid, citris acid and similar ones.

The content of pomegranate extract ranges from 200 to 1000. mg/Kg. (such pomegranate extract having a punicalagins content of 50 % w/w).

### Measurement of antioxidant capacity was carried out as follows.

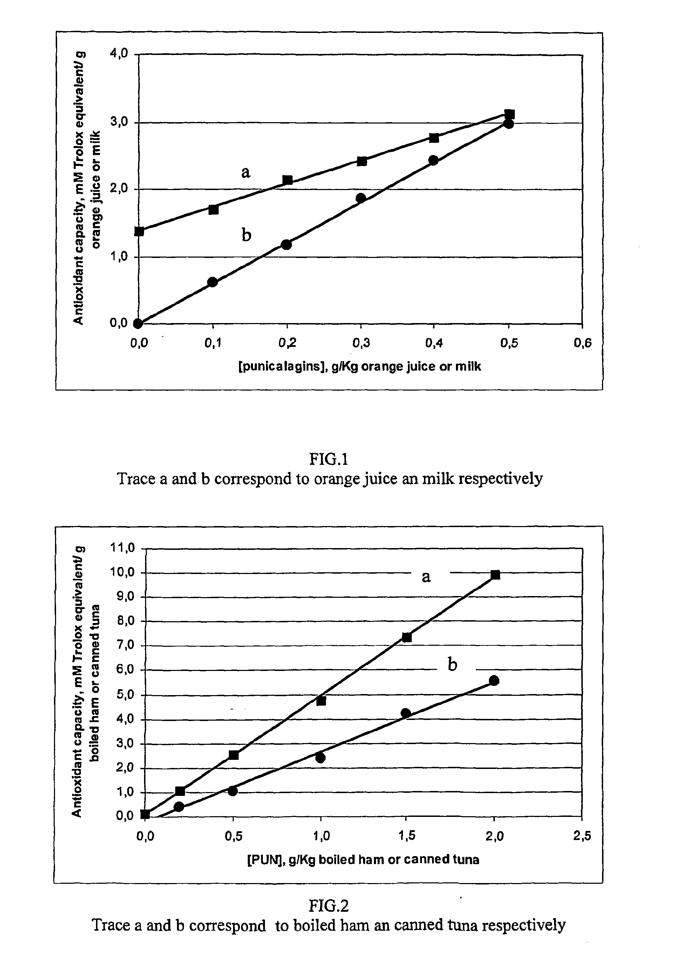

Six glass bottles of orange juice were prepared as above comprising an amount of 0, 200, 400, 600, 800 and 1000 ppm of pomegranate extract respectively (such pomegranate extract having a punicalagins content of 50% w/w ). 40 grams samples of orange juice were used to measure antioxidant capacity (radical ABTS absorption capacity measured at 734 nm using Trolox as standard) and punicalagins content by HPLC. When results were plotted (see figure 1, plot a) as antioxidant capacity vs. concentration of punicalagins a linear correlation was obtained. Antioxidant capacity of the non-functionalized juice was 1.39 mM Trolox equivalent per gram and antioxidant capacity of the functional juice with the highest weight ratio used in this example was 3.13 mM Trolox equivalent per gram, meaning that a 2.3 fold increase in the antioxidant capacity of the juice was obtained.

### EXAMPLE 2

### Preparation of a functional milk based product with pomegranate extract

Pomegranate extract was added to liquid cow milk and homogenised in the absence of oxygen. The resulting dairy product was then subjected to U.H.T. treatment (150 °C for 4 to 6 seconds) and finally packaged in the absence of oxygen.

The content of pomegranate extract ranges from 200 to 1000 mg/Kg. (such pomegranate extract having a punicalagins content of 50 % w/w).

Measurement of antioxidant capacity was carried out as follows.

Six glass bottles of milk were prepared as above comprising an amount of 0, 200, 400, 600, 800 and 1000 ppm of pomegranate extract respectively (such pomegranate extract having a punicalagins content of 50 % w/w ). 40 grams samples of milk were used to measure antioxidant capacity (radical ABTS absorption capacity measured at 734 nm using Trolox as standard) and punicalagins content by HPLC. When results were plotted (see figure 1, plot b) as antioxidant capacity vs. concentration of punicalagins a linear correlation was obtained. Antioxidant capacity of the non-functionalized milk based product was 0 mM Trolox equivalent per gram and antioxidant capacity of the functional milk based product with the highest weight ratio used in this example was 2.97 mM Trolox equivalent per gram.

### EXAMPLE 3

### Preparation of a functional boiled ham with pomegranate extract

Pomegranate extract is previously blended with the mixture of industrial ingredients (salt, glucose syrup, sugar, several food additives) and this mixture is incorporated into the brine solution. Straight afterwards the brine solution is forced through holes into the ham piece. Several massages, under vacuum and at temperature below 10 °C, are made to the ham piece. Then, ham piece is macerated at 4-6 °C during at least 48 h. After that piece is again massaged and packed under vacuum into a cooking bag that is put into a mould. The ham piece is then baked in a steam oven until the temperature in the centre of the piece reaches 65 °C. Straight afterwards the piece of boiled ham is cooled and stored at 5 °C. Finally after 24 h the mould is removed.

The content of pomegranate extract ranges from 400 to 4000 mg/Kg. (such pomegranate extract having a punicalagins content of 50 % w/w).

Measurement of antioxidant capacity was carried out as follows.

Six boiled hams were prepared as above comprising an amount of 0, 400, 1000, 2000, 3000 and 4000 ppm of pomegranate extract respectively (such pomegranate extract having a punicalagins content of 50 % w/w ). 3 grams samples of each boiled ham were used to measure antioxidant capacity (radical ABTS absorption capacity measured at 734 nm using Trolox as standard) and punicalagins content by HPLC. When results were plotted (see figure 2, plot a) as antioxidant capacity vs. concentration of punicalagins a linear correlation was obtained. Concretely antioxidant capacity of the non-functionalized boiled ham was 0.11 mM Trolox equivalent per gram and antioxidant capacity of the boiled ham with the highest weight ratio used in this example was 9.93 mM Trolox equivalent per gram, meaning that a 90.3 fold increase in the antioxidant capacity of the boiled ham was obtained.

### EXAMPLE 4

### Preparation of a functional canned tuna with pomegranate extract

Pomegranate extract is added as early as possible in the production process to facilitate its incorporation and homogeneous distribution whether the ingredients are pre-mixed with other dry ingredients or dispersed in water, according to the known art.

The content of pomegranate extract ranges from 400 to 4000 mg/Kg. (such pomegranate extract having a punicalagins content of 50 % w/w).

Measurement of antioxidant capacity was carried out as follows.

Six cans of tuna were prepared as above comprising an amount of 0, 400, 1000, 2000, 3000 and 4000 ppm of pomegranate extract respectively (such pomegranate extract having a punicalagins content of 50 % w/w ). 3 grams samples of each tuna were used to measure antioxidant capacity (radical ABTS absorption capacity measured at 734 nm using Trolox as standard) and punicalagins content by HPLC. When results were plotted (see figure 2, plot b) as antioxidant capacity vs. concentration of punicalagins a linear correlation was obtained. Concretely antioxidant capacity of the non-functionalized canned tuna was 0 mM Trolox equivalent per gram and antioxidant capacity of the canned tuna with the highest weight ratio used in this example was 5.54 mM Trolox equivalent per gram.

### EXAMPLE 5

### Preparation of a functional semi-moist dog food with pomegranate extract

Pomegranate extract is added as early as possible in the production process to facilitate its incorporation and homogeneous distribution whether the ingredients are pre-mixed with other dry ingredients or dispersed in water, according to the known art.

The content of pomegranate extract ranges from 400 to 5000. mg/Kg. (such pomegranate extract having a punicalagins content of 50 % w/w).

Measurement of antioxidant capacity was carried out as follows.

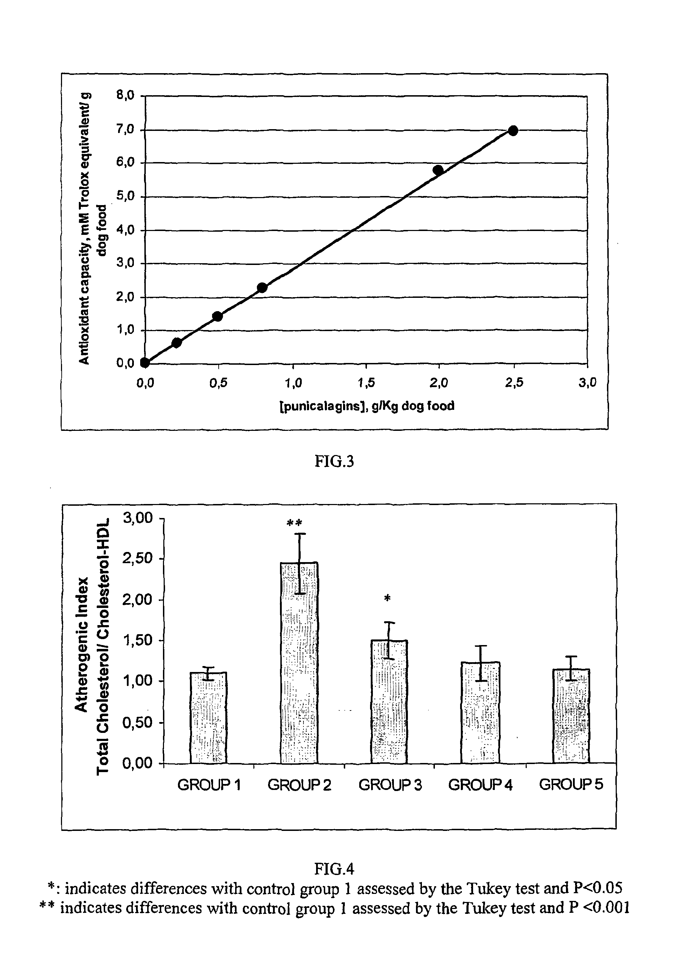

Six cans of semi-moist dog food were prepared as above comprising an amount of 0, 433, 1000, 1600, 4000 and 5000 ppm of pomegranate extract respectively (such pomegranate extract having a punicalagins content of 50 % w/w). 3 grams samples of each semi-moist dog food were used to measure antioxidant capacity (radical ABTS absorption capacity measured at 734 nm using Trolox as standard) and punicalagins content by HPLC. When results were plotted (see figure 3) as antioxidant capacity vs. concentration of punicalagins a linear correlation was obtained. Concretely antioxidant capacity of the non-functionalized dog food was 0.02 mM Trolox equivalent per gram and antioxidant capacity of the dog food with the highest weight ratio used in this example was 6.95 mM Trolox equivalent per gram, meaning that a 347.5 fold increase in the antioxidant capacity of the dog food was obtained.

### EXAMPLE 6 (fig.4)

### Use of pomegranate extract in the prevention or treatment of cardiovascular diseases.

### 6.1. Animals

Forty male Sprage Dawley rats (weight aprox. 150-180 g) were obtained from Harlan Interfauna Iberica SA (Barcelona, Spain) and maintained during all the experiment in the installations of the animalary service at the University of Murcia. The animals were randomly distributed into 5 experimental groups of 8 rats each, and every 4 rats subgroup housed under standard conditions of lighting (day/night cycles of 12 h), temperature (22±2 °C) and humidity (60%).

### 6.2. Diets

The diets used in the study were as follows:
- CONTROL DIET (C): solid standard rat diet (Panlab).
- ATHEROGENIC DIET (A): 95.5% standard rat diet (Panlab), 1.5% of cholesterol (Aldrich) and 3% of lard to induce atherosclerosis.
- ATHEROGENIC DIET + POMEGRANATE EXTRACT (P): 95.0% standard rat diet (Panlab), 1.5% of cholesterol (Aldrich), 3% of lard and 0.5 % pomegranate extract (punicalagins content: 50 % w/w).
- STANDARD + ORALLY GAVAGE (160 mg punicalagins/Kg) (G160): standard rat diet (Panlab). Additionally, 160 milligram of punicalagins/kg of body weight were administered in water by oral gavages every day.

Drinking water and food were available ad libitum. Nevertheless, throughout the study and average food supply for animal was standardised to 15 g/day (real food intake per animal was unknown). Additionally, 160 mg of punicalagins/kg of body weight, were administered in water by oral gavages to animal group receiving diet G160, every day. To avoid fat oxidation, atherogenic diet (A) and atherogenic diet + pomegranate extract (P) were kept at 4°C in the dark until use, and the non-consumed diet of past day was removed. The concentration of punicalagins in the P, and G160 diets used in the study was measured by HPLC.

### 6.3. Experimental design

The experimental design is shown in the following Table 1:

**Table 1.**

| GROUP | From day 1 to 30 | From day 31 to 60 | Remarks |
|---|---|---|---|
| 1 | C | C | |
| 2 | A | A | |
| 3 | A | C | |
| 4 | P | P | |
| 5 | A | Standard Diet + G160 | 160 mg of punicalagins per kg of BW were administered in water daily by oral gavages. |

Animal groups CC, AA, AC, PP, AG160 were fed for 2-months with diets C, A or P (groups CC, AA, or PP respectively), or for 1-month with the A-diet followed by a further month with diet C (group AC), or standard rat diet with orally gavage of 160 mg of punicalagins/kg of body weight, (group AG160). At the end of the experiment, fasted animals were anesthetized and immediately after euthanasia intra-cardiac punction was made to collect blood in tubes and 3.8 % sodium citrate was added to each tube. Plasma was separated by centrifugation for analysis.

### 6.4. Determination of lipids, in plasma.

The plasma concentrations of total cholesterol (TC), HDL-cholesterol (HDL-C) and triglycerides (TG) were determined by colorimetry using commercial kits purchased from Biosystems (Barcelona, Spain) according to the manufacturer's instructions.

### 6.5. Statistical analysis

The data are expressed (in figure 4) as means±standard error of the means (S.E.M.), and were analyzed by one-way ANOVA. Differences between the groups were assessed by the Tukey test. Differences were considered significant when P-values were <0.05. The data was analysed using Sigma Stat software (Version 2.03).

### 6.6. Discussion.

Figure 4 shows the correlation between the atherogenic index and the animals, divided according to the different diet schemes. Animal group 1 treated with CC diet (control diet for two months) shows the lowest atherogenic index value, while animal group 2, treated with AA diet (atherogenic diet - negative control for two months) shows the highest, being statistically significant the difference of the atherogenic index value between groups 1 and 2 with a P-value < 0.001. An interesting result is shown according to group 5, animals were treated with A and G160 diets during the 1^{st} and 2^{nd} month respectively. No statistically significant difference of the atherogenic index value between groups 5 and 1 was observed. In other words, after the month of treatment with the G160 diet a significant reduction of the atherogenic index value to a value that is comparable to the level obtained with CC diet (control diet for two months), was observed for animal group 5. On the contrary, the same effect was not observed when only standard diet was given in the month following the month of treatment with the atherogenic diet (animal group 3). In this case, atherogenic index value (even though being statistically significant the difference with those of negative control group 2), resulted in a statistically significant difference with respect to the atherogenic index value detected for the animals fed for two months with the control diet (group 1) with a P-value < 0.05.

High cholesterol/trilglycerides levels, low HDL cholesterol levels and specially oxidised LDLs are among risk factors associated with atherosclerosis and coronary heart disease. We have studied the effect that a functionalized nutritional product containing a pomegranate extract has on biomarkers for cardiovascular events, and compared it with similar nutritional products no-functionalized with pomegranate extract. Taking all the parameters measured into account (see figure 4), we conclude the regular administration of the nutritional product containing a pomegranate extract protects the cardiovascular system which, to a greater or lesser extent, prevents the occurrence of cardiovascular events and therefore may well be considered as a health promoting nutritional product.

Especially remarkable is the fact that addition of pomegranate extracts to a fat and cholesterol rich diet (PP, group 4) permits to maintain the value of atherogenic index without a statistically significant difference when compared with a standard diet (CC, group 1).

### EXAMPLE 7 (fig.5)

### Use of pomegranate extract in the treatment of hypertension.

### 7.1. Animals

Twenty-four male Sprage Dawley rats (weight aprox. 200 g at the init of the experiment) were obtained from Harlan Interfauna Iberica SA (Barcelona, Spain) and maintained during all the experiment in the installations of the animalary service at the University of Murcia. The animals were randomly distributed into four experimental groups of 6 rats each one, housed under standard conditions of lighting (day/night cycles of 12 h), temperature (22±2 °C) and humidity (60%).

### 7.2. Diet

During the acclimatation and experimental periods, rats were fed on a solid standard diet for rats (Panlab). Drinking water and food were available ad libitum. After an acclimatation period of 7 days treatments according the experimental design were started.

### 7.3. Experimental design

In order to determine effect of pomegranate extract an N-nitro-L-arginine-methylester (L-NAME) induced hypertension model was used. After the acclimatation period, but before the L-NAME induced hypertension treatment was started, basal recordings of arterial blood pressure (D0) were measured with a Letica 5002 equipment.

Animals were treated according experimental design as showed in the following table:

**Table 2.**

| GROUP | Drinking water with L-NAME * | Oral gavages | Remarks |
|---|---|---|---|
| 1 | No | No | |
| 2 | 40 mg L-NAME/Kg | No | |
| 3 | 40 mg L-NAME/Kg | 160 mg punicalagins/ Kg | punicalagins into water |
| 4 | 40 mg L-NAME/Kg | 100 mg Captopril/ Kg | Positive control: 100 mg of captopril (dissolved in water) |

| | | | |
|---|---|---|---|
| *: L-NAME treatment was administered to all groups via the drinking water, available ad libitum, taking into consideration a body weight of. 200 g per animal and standardising the average drinking water supply for animal to 40 mL/day (real drinking water intake per animal was unknown) | | | |

### 7.4. Time table of activities

In order to achieve rats to be in the habit, measurements of arterial blood pressure using the recording of the pulsations of the tail artery with a Letica 5002 equipment were made during the acclimatation period.

The carrying out of experimental measurements/treatments were summarized in the following table:

**Table 3.**

| Day | Measurements of arterial blood pressure | Drinking water supplemented with L-NAME | Oral gavages |
|---|---|---|---|
| 0 | Yes | Yes | No |
| 1 | No | Yes | Yes |
| 2 | Yes | Yes | Yes |
| 3 | No | Yes | Yes |
| 4 | Yes | Yes | Yes |
| 5 | No | Yes | Yes |
| 6 | No | Yes | Yes |
| 7 | Yes | Yes | Yes |

### 7.4. Determination of arterial blood pressure.

Arterial blood pressure was measured with a periodicity according the above table, by the tail-cuff method. Before the measurement, the rats were kept at 37 °C for 10 min to make the pulsations of the tail artery detectable. The equipment used in the present study, LE 5002 (Letica, Hospitalet, Barcelona, Spain), has a high sensitivity pulse transducer coupled with an accurate microprocessor program, and allow us accurate measurements of arterial blood pressure. The arterial blood pressure measurements were performed at the same time of the day in order to avoid any influence of the circadian cycle.

### 7.5. Statistical analysis

The data are expressed (see figure 5) as means±standard error of the means (S.E.M.), and were analyzed using Sigma Stat-software (Version 2.03).

### 7.6. Discussion.

Arterial hypertension is among risk factors associated with cardiovascular diseases. We have studied the effect that pomegranate extract has on arterial blood pressure, and compared it with control groups without treatment with pomegranate extract. We conclude that pomegranate extract showed antihypertensive activity which, to a greater or lesser extent, prevents the occurrence of cardiovascular events and therefore may well be considered as a health promoting dietary supplement and may be a successful strategy to produce functional foods and beverages with antihypertensive activity.

### EXAMPLE 8a (fig.6)

### Use of pomegranate extract and soluble fiber in the prevention or treatment of cardiovascular diseases.

### 8. 1. Animals

Seventy two male Sprage Dawley rats (weight aprox. 150-180 g) were obtained from Harlan Interfauna Iberica SA (Barcelona, Spain) and maintained during all the experiment in the installations of the animalary service at the University of Murcia. The animals were randomly distributed into 9 experimental groups of 8 rats each, and every 4 rats subgroup housed under standard conditions of fighting (day/night cycles of 12 h), temperature (22±2 °C) and humidity (60%).

### 8.2. Diets

The diets used in the study were as follows:
- CONTROL DIET (C): solid standard rat diet (Panlab).
- ATHEROGENIC DIET (A): 95.5% standard rat diet (Panlab), 1.5% of cholesterol (Aldrich) and 3% of lard to induce atherosclerosis.
- STANDARD + ORALLY GAVAGE (40 mg punicalagins/Kg) (G40): standard rat diet (Panlab). Additionally, 40 milligram of punicalagins/kg of body weight were administered in water by oral gavages every day.
- SOLUBLE FIBER DIET (F): 95% standard rat diet (Panlab) and 5% of soluble fiber.
- SOLUBLE FIBER + GAVAGE (40 mg punicalagins / Kg) (FG): 95% standard rat diet (Panlab) and 5% of soluble fiber. Additionally, 160 milligram of punicalagins/kg of body weight, in water, were administered by oral gavages every day.
- ATHEROGENIC DIET + ORALLY GAVAGE (40 mg punicalagins/Kg) (AG): 95.5% standard rat diet (Panlab), 1.5% of cholesterol (Aldrich), 3% of lard. Additionally, 40 milligram of punicalagins/kg of body weight were administered in water by oral gavages every day
- ATHEROGENIC DIET + SOLUBLE FIBER (AF): 90.5% standard rat diet (Panlab), 1.5% of cholesterol (Aldrich) and 3% of lard to induce atherosclerosis and 5% soluble fiber.
- ATHEROGENIC DIET + SOLUBLE FIBER + GAVAGE (40 mg punicalagins / Kg) (AFG): 90.5% standard rat diet (Panlab), 1.5% of cholesterol (Aldrich) and 3% of lard to induce atherosclerosis and 5% soluble fiber. Additionally, 40 milligram of punicalagins/kg of body weight, in water, were administered by oral gavages every day.

Drinking water and food were available ad libitum. Nevertheless, throughout the study the average food supply for animal was standardised to 15 g/day (real food intake per animal was unknown). Additionally, 40 mg of punicalagins/kg of body weight was administered by oral gavages to animal groups G40, FG, AG and AFG respectively, every day. To avoid fat oxidation, atherogenic diet was kept at 4°C in the dark until use, and the non-consumed diet of past day was removed.

The concentrations of punicalagins in G40, FG, AG and AFG was measured by high performance liquid chromatography (HPLC).

### 8.3. Experimental design

The experimental design is shown in the following Table 4:

**Table 4.**

| GROUP | From day 1 to 30 | From day 31 to 60 | Remarks |
|---|---|---|---|
| 1 | C | C | |
| 2 | A | A | |
| 3 | A | C | |
| 4 | A | F | |
| 5 | A | G40 | 40 mg of punicalagins per kg of BW, in water, were administered daily by oral gavages. |
| 6 | A | FG | 40 mg of punicalagins per kg of BW, in water, were administered daily by oral gavages. |
| 7 | AF | AF | |
| 8 | AG | AG | 40 mg of punicalagins per kg of BW, in water, were administered daily by oral gavages. |
| 9 | AFG | AFG | 40 mg of punicalagins per kg of BW, in water, were administered daily by oral gavages. |

Animal groups, were fed for 2-months with diets C, A, AF, AG or AFG (groups 1, 2, 7, 8 and 9 respectively), or for 1-month with the A-diet followed by a further month with diet C, F, G40 or FG (group 3, 4, 5 and 6 respectively). At the end of the experiment, fasted animals were anesthetized and immediately after euthanasia intra-cardiac punction was made to collect blood in tubes. Serum was separated by centrifugation for analysis.

### 8.4. Statistical analysis

The data related with the atherogenic index, are expressed as means±standard error of the means (S.E.M.), and were analyzed by one-way ANOVA. Differences between the groups were assessed by the Tukey test. Differences were considered significant when P-values were <0.05. The data was analysed using Sigma Stat software (Version 2.03).

The data related with the rat BW are expressed as means±standard error of the means (S.E.M.), and were analyzed using Sigma Stat software (Version 2.03).

### 8.5. Discussion.

The obtained results are shown in the enclosed figure 6, where the atherogenic index has been correlated with different animal groups that, as already said, are indicated in Table 4.

Figure 6 shows the correlation between the atherogenic index and the animals, divided according to the different diet schemes. Animal group 1 treated with CC diet (control diet for two months) shows the lowest atherogenic index value, while animal group 2, treated with AA diet (atherogenic diet - negative control for two months) shows the highest, being statistically significant the difference of the atherogenic index value between groups 1 and 2 with a P-value < 0.001. An interesting result is shown according to group 6, animals were treated with A and FG diets during the 1^{st} and 2^{nd} month respectively. No statistically significant difference of the atherogenic index value between groups 6 and 1 was observed. In other words, after the month of treatment with the FG diet a significant reduction of the atherogenic index value (a 30,2 % reduction over the average value obtained for group 3, treated with diet A-C) to a value that is comparable to the level obtained with CC diet (control diet for two months), was observed for animal group 6. On the contrary, the same effect was not observed when only soluble fiber (Diet F) or a gavage of 40mg pomegranate extract/Kg BW (Diet G40) were given to the animals in addition to a standard diet in the month following the month of treatment with the atherogenic diet (animal group 4 and group 5 respectively). In this case, atherogenic index value (even though being statistically significant the difference with those of negative control group 2), resulted in a statistically significant difference with respect to the atherogenic index value detected for the animals fed for two months with the control diet (group 1) with a P-value < 0.05. Surprisingly, the reduction of the atherogenic index value over the average value obtained for the group 3, treated with diet A-C, were 6,9 % and 17,2 % respectively for the groups 4 and 5 respectively, suggesting, when compared with the 30,2 % reduction obtained for the group 6, a surprising, and possibly synergic, effect due to the combination of soluble fiber and pomegranate fruit extract according to the present invention.

Another interesting result is shown according to animal group 9, that, in this experiment, were treated with AFG diet. No statistically significant difference of the atherogenic index value between group 9 and 1 was observed. In other words, after two month of treatment with the atherogenic diet supplemented with soluble fiber plus a gavage of pomegranate fruit extract rich in punicalagins, an unaltered atherogenic index value that is comparable to the level obtained with the control diet, was observed for animal group AFG-AFG. On the contrary, the same effect was not observed when only AF or AG diets were given to the animals of groups 7 and 8 respectively during the two months of experiment (animal groups AF-AF and AG-AG). In this case, atherogenic index value (even though being statistically significant the difference with those of negative control group 2), resulted in a statistically significant difference with respect to the atherogenic index value detected for the animals fed with the control diet (group 1) with a P-value < 0.05. The atherogenic index level derived from animal group 9 suggest a preventive effect due to the pomegranate fruit extract rich on punicalagins obtained according to the present invention and suggest a improvement of the effect due to the combination of soluble fiber and pomegranate fruit extract according to the present invention.

### EXAMPLE 8b (fig.7)

Obesity is among risk factors associated with metabolic syndrome and people with metabolic syndrome have an increased risk of cardiovascular diseases. We have studied the effect that pomegranate extract in combination with soluble fiber has on rat body weight (BW), and compared it with control groups without treatment with the combination of soluble fiber and pomegranate fruit extract according to the present invention.

Figure 7 shows the correlation between the BW of the animals, same used in example 8a, divided according to the different diet schemes and following the experimental design shown in the Table 4 of the previous example 8a..

According to said Figure 7, after two months of experiment, animal group 1 treated with CC diet (control diet) shows the lowest BW, while animal group 2, treated with AA diet (atherogenic diet- negative control) shows the highest BW. The atherogenic diet and total time of the experiment were selected in order to avoid obesity in the animals that could affect the correct interpretation of the data obtained for plasma concentrations of total cholesterol (TC), HDL-cholesterol (HDL-C) and triglycerides.

An interesting result is shown according to animals of group 9, that, in this experiment, were treated with AFG diet. Similar trends of rat BW value during the two months experiment between group 9 and 1 were observed. In other words, after two month of treatment with the atherogenic diet supplemented with soluble fiber plus a gavage of pomegranate fruit extract rich in punicalagins, a rat BW comparable to the value obtained with the control diet, was observed for animal group AFG-AFG. By the contrary, the same effect was not observed when only an AF or AG diets given to the animals of groups 7 and 8 respectively during the two months of experiment (animal group AF-AF). In this case, trends of rat BW of groups 7 and 8 appear and intermediate situation between the highest rat BW trend, group 2, and the lowest rat BW trend, group 1. The BW trend shown by animal group 9 suggest a preventive effect on overweight/obesity due to combination of pomegranate fruit extract and soluble fiber according to the present invention.

Taking all the measured parameters into account, it can be concluded that the regular administration of the pomegranate extract in combination with soluble fiber according to the present invention, protects the cardiovascular system which, to a greater or lesser extent, prevents the occurrence of some of the risk factors that are considered associated with atherosclerotic plaque builds up in the arteries and metabolic syndrome, and therefore may well be considered a health promoting natural extract, to be used in the preparation of functional foods and dietary supplements.

## Claims

1. The use of a pomegranate extract comprising punicalagins, ellagic acid and sugars, wherein the ratio (w/w%) punicalagin/ellagic acid is in the range of 10/1 to 35/1, the ratio (w/w%) punicalagin/sugars is within the range of 10/1 to 50/1, the total sugar content is 2 % (w/w) or less and wherein said extract has a punicalagins content of at least 30 % w/w, an ellagic acid content of 2 % (w/w) or less, a total phenols content of at least 30 % w/w, (expressed as gallic acid equivalent), a water solubility of at least 7 % w/v (70 g per litre) and a residual organic solvent content of less than 1 ppb for preparing a nutritional product.

2. The use of claim 1, wherein said pomegranate extract has a water solubility of at least 10 % w/v (100 g per litre) and a total sugar content of not more than 1 % (w/w).

3. The use of claim 1 or 2, wherein said pomegranate extract comprises a soluble fiber having a beta-glucan (dry basis) content of at least 30 %.

4. A nutritional product which is a functional food or functional beverage comprising a pomegranate extract as defined in any claim 1 to 3, **characterized in that** in said product the ratio (w/w%) punicalagin/ellagic acid is in the range of 10/1 to 35/1 and the ratio (w/w%) punicalagin/added pomegranate sugars is within the range of 10/1 to 50/1.

5. A nutritional product according to claim 4, comprising fructose, wherein the ratio (w/w%) punicalagin/fructose in the said nutritional product is in the range of 60/1 to 100/1.

6. A nutritional product according any claim 4 to 5, comprising soluble fibers.

7. A nutritional product according any claim 4 to 6, **characterized in** comprising a punicalagins content within the range of 0.005% to 5% w/w.

8. A nutritional product according to any claim 4 to 7, further comprising hyd roxytyrosol.

9. A nutritional product according to any claim 4 to 8, wherein said product is selected from a drink, a beverage, a dairy product; a vegetable, marine or fish edible oil; a meat or poultry product; a bakery product or a pasta-based product; a vegetable or fruit conserve; a canned seafood or canned fish; a snack, a sweet, salt, sugar, a seasoning or combinations of two or more thereof.

10. A nutritional product according to any claim 4 to 9, which is a pet or animal functional food.

11. The use of the extract as defined in any claim 1 to 3 as food additive to prevent or inhibit lipid oxidation of food and beverages.

12. The use of the extract as defined in any claim 1 to 3 as food additive to prevent or inhibit spoilage of food and beverages due to fungi, bacteria and other microorganisms.

13. An extract as defined in any claim 1 to 3 for use in the prevention or treatment of one or more conditions selected from cardiovascular diseases, arterial hypertension, plaque build-up in the arteries and metabolic syndrome.

14. A nutritional product according to any claim 4 to 10 for use in the prevention or treatment of one or more conditions selected from cardiovascular diseases, arterial hypertension, plaque build-up in the arteries and metabolic syndrome.

15. The use of an extract as defined in any claim 1 to 3 for the manufacture of a medicament and/or a composition for the prevention or treatment of one or more conditions selected from cardiovascular diseases, arterial hypertension, plaque build-up in the arteries and metabolic syndrome.

## Patentansprüche

1. Verwendung eines Granatapfelextraktes umfassend Punicalagine, Ellagsäure und Zucker, wobei das Verhältnis (Gew./Gew.-%) Punicalagin/Ellagsäure im Bereich von 10/1 bis 35/1 liegt, das Verhältnis (Gew./Gew.-%) Punicalagin/Zucker innerhalb des Bereiches von 10/1 bis 50/1 liegt, der Gesamtzuckergehalt beträgt 2% (Gew./Gew.) oder weniger und wobei das genannte Extrakt einen Punicalagingehalt von mindestens 30 Gew./Gew.-%, einen Ellagsäuregehalt von 2% (Gew./Gew.) oder weniger, einen Gesamtphenolgehalt von mindestens 30 Gew./Gew.-% (ausgedrückt als Gallussäure-Äquivalent), eine Wasserlöslichkeit von mindestens 7 Gew./Vol.-% (70 g pro Liter) und einen Restgehalt an organischen Lösungsmitteln von weniger als 1 ppb aufweist, zur Zubereitung eines Ernährungsprodukts.

2. Verwendung nach Anspruch 1, wobei das genannte Granatapfelextrakt eine Wasserlöslichkeit von mindestens 10 Gew.Nol.-% (100 g pro Liter) und einen Gesamtzuckergehalt von nicht mehr als 1% (Gew./Gew.) aufweist.

3. Verwendung nach Anspruch 1 oder 2, wobei das genannte Granatapfelextrakt eine lösliche Faser mit einem Beta-Glucangehalt (Trockenbasis) von mindestens 30% umfasst.

4. Ernährungsprodukt das ein funktionelles Lebensmittel oder ein funktionelles Getränk ist, umfassend ein Granatapfelextrakt nach einem der Ansprüche 1 bis 3, **dadurch gekennzeichnet, dass** im genannten Produkt das Verhältnis (Gew./Gew.-%) Punicalagin/Ellagsäure im Bereich von 10/1 bis 35/1 liegt und das Verhältnis (Gew./Gew.-%) Punicalagin/zugesetzter Granatapfelzucker innerhalb des Bereiches von 10/1 bis 50/1 liegt.

5. Ernährungsprodukt nach Anspruch 4, umfassend Fruktose, wobei das Verhältnis (Gew./Gew.-%) Punicalagin/Fruktose im genannten Ernährungsprodukt im Bereich von 60/1 bis 100/1 liegt.

6. Ernährungsprodukt nach einem der Ansprüche 4 bis 5, umfassend lösliche Fasern.

7. Ernährungsprodukt nach einem der Ansprüche 4 bis 6, **dadurch gekennzeichnet, dass** es einen Punicalagingehalt innerhalb des Bereiches von 0,0005 Gew./Gew.-% bis 5 Gew./Gew.-% umfasst.

8. Ernährungsprodukt nach einem der Ansprüche 4 bis 7, zusätzlich umfassend Hydroxytyrosol.

9. Ernährungsprodukt nach einem der Ansprüche 4 bis 8, wobei das genannte Produkt aus einem Erfrischungsgetränk, einem Getränk, einem Milchprodukt; einem pflanzlichen Öl, Öl aus Fischen oder Meeressäugetieren für Speisezwecke; einem Fleisch- oder Geflügelprodukt; einem Bäckereiprodukt oder einem auf Pasta basierten Produkt; einer Gemüse- oder Obstkonserve; einer Meeresfrüchtekonserve oder Fischkonserve; einem Snack, einer Süßigkeit, Salz, Zucker, einem Gewürz oder Kombinationen von zwei oder mehr derselben, ausgewählt wird.

10. Ernährungsprodukt nach einem der Ansprüche 4 bis 9, das ein funktionelles Lebensmittel für Tiere oder Haustiere ist.

11. Verwendung des Extrakts nach einem der Ansprüche 1 bis 3 als Lebensmittelzusatz um die Lipidoxidation von Lebensmitteln und Getränken vorzubeugen oder zu hemmen.

12. Verwendung des Extraktes nach einem der Ansprüche 1 bis 3 als Lebensmittelzusatz um die Verderbung von Lebensmitteln und Getränken aufgrund Pilze, Bakterien und anderer Mikroorganismen vorzubeugen oder zu hemmen.

13. Extrakt nach einem der Ansprüche 1 bis 3 für dessen Verwendung bei der Vorbeugung oder Behandlung einer oder mehreren Erkrankungen ausgewählt aus Herz-Kreislauf-Erkrankungen, Bluthochdruck, Ansammlung von Plaque in den Arterien und Stoffwechselsyndrom.

14. Ernährungsprodukt nach einem der Ansprüche 4 bis 10 für dessen Verwendung bei der Vorbeugung oder Behandlung einer oder mehreren Erkrankungen ausgewählt aus Herz-Kreislauf-Erkrankungen, Bluthochdruck, Ansammlung von Plaque in den Arterien und Stoffwechselsyndrom.

15. Verwendung eines Extraktes nach einem der Ansprüche 1 bis 3 für die Herstellung eines Medikaments und/oder einer Zusammensetzung zur Vorbeugung oder Behandlung einer oder mehreren Erkrankungen ausgewählt aus Herz-Kreislauf-Erkrankungen, Bluthochdruck, Ansammlung von Plaque in den Arterien und Stoffwechselsyndrom.

## Revendications

1. L'utilisation d'extrait de grenade comprenant des punicalagins, de l'acide ellagique et des sucres, dans laquelle le rapport (p/p%) de punicalagin/acide ellagique se situe entre 10/1 et 35/1, le rapport (p/p%) de punicalagin/sucres se situe entre 10/1 et 50/1, la teneur totale en sucre est de 2% (p/p) ou inférieure et dans laquelle cet extrait a une teneur en punicalagins d'au moins 30% p/p, une teneur en acide ellagique de 2% (p/p) ou inférieure, une teneur en phénols totale d'au moins 30% p/p, (exprimée comme équivalent à l'acide gallique), une hydrosolubilité d'au moins 7% p/v (70 g par litre) et une teneur en dissolvant organique résiduel d'au moins 1 ppb pour préparer une produit nutritionnel.

2. L'utilisation de la revendication 1, dans laquelle cet extrait de grenade a une hydrosolubilité d'au moins 10% p/v (100 g par litre) et une teneur totale en sucre non supérieure à 1 % (p/p).

3. L'utilisation de la revendication 1 ou 2, dans laquelle cet extrait de grenade comprend une fibre soluble ayant une teneur en bêta-glucane (base sèche) d'au moins 30%.

4. Un produit nutritionnel étant un aliment fonctionnel ou une boisson fonctionnelle comprenant un extrait de grenade tel qu'il est défini dans une quelconque des revendications 1 à 3, **caractérisé en ce que** dans ce produit le rapport (p/p%) de punicalagin/acide ellagique se situe entre 10/1 et 35/1 et le rapport (p/p%) de punicalagin/sucres de grenade ajoutés se situe ente 10/1 et 50/1.

5. Un produit nutritionnel conformément à la revendication 4, comprenant de la fructose, dans lequel le rapport (p/p%) de punicalagin/fructose dans ce produit nutritionnel se situe entre 60/1 et 100/1.

6. Un produit nutritionnel conformément à une quelconque des revendications 4 à 5, comprenant des fibres solubles.

7. Un produit nutritionnel conformément à une quelconque des revendications 4 à 6, **caractérisé en ce qu'**il comprend une teneur en punicalagins qui se situe entre 0,005% et 5% p/p.

8. Un produit nutritionnel conformément à une quelconque des revendications 4 à 7, comprenant de plus de l'hydroxytyrosol.

9. Un produit nutritionnel conformément à une quelconque des revendications 4 à 8, dans lequel ce produit a été choisi parmi des boissons alcoolisées et non alcoolisés, un produit laitier; un légume, une huile marine ou une huile de poisson comestible; de la viande ou de la volaille; un produit de boulangerie ou un produit à base de pâtes; une conserve végétale ou un fruit en conserve; des fruits de mer ou du poisson en conserve; une collation, une confiserie, du sel, du sucre, un assaisonnement ou bien une combinaison de deux ou plus d'entre eux.

10. Un produit nutritionnel conformément à une quelconque des revendications 4 à 9, qui est un aliment fonctionnel pour animaux de compagnie ou autres.

11. L'utilisation de l'extrait tel qu'elle est définie dans une quelconque des revendications 1 à 3 comme additif alimentaire pour éviter ou empêcher l'oxydation des lipides des aliments ou boissons.

12. L'utilisation de l'extrait tel qu'elle est définie dans une quelconque des revendications 1 à 3 comme additif alimentaire pour éviter ou empêcher que les aliments ou boissons ne s'abîment à cause d'un fongus, une bactérie et autres micro-organismes.

13. Un extrait tel qu'il est défini dans une quelconque des revendications 1 à 3 pour son utilisation dans la prévention ou traitement d'une ou plusieurs maladies parmi les cardiovasculaires, l'hypertension artérielle, la formation de plaques dans les artères et le syndrome métabolique.

14. Un produit nutritionnel tel qu'il est défini dans une quelconque des revendications 4 à 10 pour son utilisation dans la prévention ou traitement d'une ou plusieurs maladies parmi les cardiovasculaires, l'hypertension artérielle, la formation de plaques dans les artères et le syndrome métabolique.

15. L'utilisation d'un extrait tel que défini dans une quelconque des revendications 1 à 3 pour la fabrication d'un médicament et/ou d'une composition pour la prévention ou le traitement d'une ou plusieurs maladies parmi les cardiovasculaires, l'hypertension artérielle, la formation de plaques dans les artères et le syndrome métabolique.
